# EUROPEAN PATENT SPECIFICATION

(11) **EP 1 603 517 B1**
(45) Date of publication and mention of the grant of the patent: **21.02.2007**
(21) Application number: 04721209.7
(22) Date of filing: 17.03.2004
(51) Int. Cl.: A61K 8/36, A61K 8/92, A61Q 19/00

(54) **SKIN CARE PRODUCT CONTAINING TALL OIL FATTY ACIDS AND VEGETABLE OILS FOR DRY AND SCALING SKIN AND TREATMENT OF PSORIASIS, DERMATITIS, AND ECZEMAS**
HAUTPFLEGEPRODUKT MIT TALLÖL-FETTSÄUREN UND PFLANZENÖLEN FÜR TROCKENE UND SCHUPPENDE HAUT UND BEHANDLUNG VON PSORIASIS, DERMATITIS UND EKZEMEN
PRODUIT DE SOIN POUR LA PEAU CONTENANT DES ACIDES GRAS DE RESINE LIQUIDE ET DES HUILES VEGETALES POUR PEAU SECHE ET DESQUAMEE ET TRAITEMENT DU PSORIASIS, DE LA DERMATITE ET DES ECZEMAS

(30) Priority: 17.03.2003 FI 20030395
(43) Date of publication of application: 14.12.2005
(73) Proprietor: Oy Psorioil Ltd., 46901 Anjalankoski (FI)
(72) Inventor: KAURANEN, Erkki, FI-45720 Kuusankoski (FI)
(74) Representative: Matilainen, Mirja Helena
(86) International application number: PCT/FI2004/000154
(87) International publication number: WO 2004/082642

(56) References cited:
- CN-A- 1 246 367
- FR-A1- 2 792 203
- GB-A- 2 090 526
- US-A- 2002 039 591
- DATABASE WPI Week200049, Derwent Publications Ltd., London, GB; Class D21, AN 2000-541769, XP002979289 & RU 2 144 351 C1 (BENCHAROV YU V) 20 January 2000
- DATABASE WPI Week199548, Derwent Publications Ltd., London, GB; Class A96, AN 1995-372553, XP002979290 & RESEARCH DISCLOSURE (HERCULES INC) vol. 378, no. 007, 10 October 1995, EMSWORTH, GB,
- DATABASE WPI Week199423, Derwent Publications Ltd., London, GB; Class A96, AN 1994-188867, XP002979291 & JP 6 128 121 A (SANSEI SAIYAKU KK) 10 May 1994
- DATABASE WPI Week200022, Derwent Publications Ltd., London, GB; AN 2000-255186, XP002979292 & RU 2 126 676 C1 (CHUBATOVA S A) 27 February 1999

## Description

### Field of the invention

The objects of the present invention are skin care products suitable for all skin types for the treatment of dry and scaling skin. The preparation according to the invention is preferably a cream or an oil, but it may also be an emulsion, gel, spray, foam, stick, roll-on, or scalp. These skin care products have been planned for use in the treatment of psoriasis. The skin care products can also be used in the treatment of the following skin diseases: dermatitis, eczemas and scale.

### Prior art

In US Patent No. 4,443,437, a composition is disclosed which is used on the skin of animals to cure wounds etc and which comprises tall oil, that is crude tall oil or products obtained by treatment of said tall oil. According to the patent, the oil comprises tall oil fatty acids and also their esters. From Chinese patent CN 1246367 a preparation is known, which comprises distilled tall oil and which is used *i*.*a*. in the treatment of psoriasis. From the publication US A1 2002/0039591, a skin care preparation is known, which comprises vegetable oil and mineral oil.

### Background of the invention

Psoriasis is a metabolic disease for which no cure has been found. Majority of psoriasis patients can be treated under non-institutional care by topical treatment and phototherapy. As topical treatment for scale peeling of psoriasis plaques, 5% salisylic vaseline (Acid. Salic. 5.0/Vaselin. Alb. Ad 100.0), which is spread on the plaques in the evenings, has been used. In the morning the agent is washed away. For the peeling of scalp scale, white vaseline is replaced for example by "Suave"- hair cream, by unguentum Macrogol DF or by castor oil. A strong or an extremely strong corticosteroid cream is suitable for a brief treatment of few psoriasis plaques.

In recurrent use, care must be taken to avoid local skin atrophy and to change early enough to alternative treatments. In prolonged and recurrent treatment, the effectiveness is weakened (tachyphylaxis) and the risk for local adverse effects is increased. A corticosteroid preparation is also suitable for the treatment of psoriasis in the scalp. Solutions, which contain alcohol, do not stain but their problem is smarting.

A corticosteroid emulsion cream or gel is at least as effective as solutions. A corticosteroid of medium strength can be used during short term on bend areas and on the face, bearing in mind, however, the risk of atrophy. During hospital care, ditranol is used for the treatment of psoriasis. Ditranol irritates the skin and stains both skin and clothes. The treatment can be made more effective by combining it with SUP or UVB phototherapy.

Tar preparations are usually not sufficient for curing psoriasis, but they can be used as intensifiers of sun or phototherapy (SUP,UVB). A tar preparation (Pyrol. lithantr. 1.0 /Ambilan ad 100.0 or a commercially available Alphosyl liniment or cream) is spread on the psoriasis plaques in the evening and washed away in the morning before going under the sun or to phototherapy. Tar shampoo can be used for the treatment of psoriasis in the scalp.

Calcipotriol is a vitamin D derivative, which probably is as effective as strong corticosteroids. Calcipotriol cream is not recommended to be used over 50 grams per week and, consequently, it cannot be used for a very large psoriasis. It is not recommended for the treatment of psoriasis of the face, either, due to skin irritation which sometimes occurs as a side effect.

Ultraviolet radiation has a clear curative effect on psoriasis. Staying under the sun (heliotherapy) cures psoriasis with very thin and small plaques and makes even rather thick plaques thinner. The effect can be intensified by peeling the scale with salisylic vaseline already before going under the sun and by using a suitable topical treatment (tar, calcipotriol) on the thicker plaques. In the treatment of the most difficult forms of psoriasis, internal medication is used. Acitretin is a retinoid or vitamin A derivative used internally for the treatment of psoriasis. Metotrexate is the most effective single medicine for psoriasis. However, it has plenty of side effects, of which the most common are nausea, abdomal pains, leukopenia and liver toxicity (Väinö Havu, Ihotaudit, 1st edition, Kustannus Oy Duodecim 1995, Helsinki).

Emulsions are widely used in cosmetics. Usually an emulsion is described as a thermodynamically labile system, which consists of at least two liquids, which are insoluble to each other and of which one is mixed as fine droplets inside the other. If the emulsion has not stabilized, the liquids immiscible to each other tend to separate.

Stabilization is achieved by adding emulsifiers to the borderline and by using, in addition, polymers, solid substances and liquid-crystal phases. For example aluminium stearate has been used as an emulsifier. Typical emulsions are water-oil emulsions (Ullmann's Encyclopedia of Industrial Chemistry, Fifth, Completely Revised Edition Volume A 24).

Greases are usually thickened with metallic soaps. Aluminium soap fats have usually been prepared from pre-made aluminium soaps, e.g. aluminium stearite, and from mineral oils (Ullmann's Encyclopedia of Industrial Chemistry, Fifth, Completely Revised Edition Volume A 15).

Swedish Bofors gun grease, Vapenfett I, is as to its type an aluminium soap grease, prepared of mineral oils. Vapenfett I does not contain vegetable oils. A lubricant is a substance, usually an oil, which is used for lubricating the machinery (The Oxford English Dictionary, Second Edition, Claredon Press, Oxford 1989). Lubricants have not been planned for use as skin creams.

Lipogels or fatty gels are most popular when a skin protecting effect is desired. Fatty gels adhere to the skin surface for a long time and produce a water-resistant effect. According to the intended purpose of use, fatty gels are prepared of semisolid fats and waxes, mixed in volatile silicone oils (*e.g.* cyclomethicone), or in colloidal swelling agents (Aerosil, bentonite), together with mineral or vegetable oils. Fatty gels are not emulsions, as they do not contain two or more liquids which are immiscible to each other. Fatty gels do not contain water as water-oil emulsions do.

Corticosteroids used nowadays for psoriasis treatment are not suitable for continuous use due to the local side effects. The white vaseline based creams used for scale peeling of psoriasis make skin dry and thus are not suitable for a healthy skin. In heliotherapy, both white vaseline and tar products are used to intensify the effect of sunlight. White vaseline and tar creams do not provide any protection against the detrimental UV radiation of sunlight. Tar preparations have to be washed away before going under the sun or to heliotherapy. Nowadays the preparations do not contain any added UV protection.

### Summary of the invention

The purpose of the invention is to provide skin care products suitable to continuous use for all skin types. The skin care products can be used for peeling and removal of scale, for the treatment of skin redness after the peeling phase, and for maintenance treatment of cured skin on healthy skin.

The effect of the skin care products which are the object of the invention, is based on the combined effect of tall oil fatty acids or their derivatives and vegetable oils. The care products can be prepared as full fat products (essential fatty acids) or for example as a water-oil emulsion and as an oil-water emulsion for maintaining moisture balance of the skin.

### Detailed description of the invention

The invention is directed to skin care products which contain tall oil fatty acids or their derivatives and one or more vegetable oils selected from the group consisting of sunflower oil, grape seed oil, rapeseed oil, tall seed oil, flaxseed oil, peanut oil, buckthorn oil, blackcurrant seed oil, siberian pine seed oil, safflor oil, primrose oil, and a mixture of two or more oils. The preparation according to the invention is preferably a cream or an oil.

A creamy or oily preparation according to the invention is intended either for topical treatment or for the whole body. With a creamy preparation is meant a product, wherein the beneficial ingredients are a mixture of fatty acids from tall oil and from different plants, to which mixture are added, if needed, for example thickeners and emulsifiers as well as moisture binding ingredients.

With an oily skin care preparation is meant a product which as to its viscosity is rather flowing, almost liquid form, but it may also be a thin emulsion. As an oil, the basis of the preparation is composed of vegetable oil(s) and tall oil fatty acids. An oily preparation is suitable for the whole body and also for the scalp, whereby alcohol derivatives or propylene glycol are used in the preparation for scale removal Skin softening vegetable oils have been added to the preparations to prevent healthy skin from drying during the treatment. When used in the scalp, the preparation removes scale and cures the skin of the scalp.

The skin treatment preparations can be provided with UV protection, which enables their use also under the sun. For example titanium dioxide can be added to the preparations according to the invention in a suitable amount, such as for example 0.1-10 % by weight.

The preparation according to the invention may also be an emulsion, gel, spray, foam, stick, roll-on, or scalp (liquid for the scalp). With an emulsion-like preparation is meant products, which include an oil-water emulsion, a water-oil emulsion, or a double emulsion. With an oil-water emulsion is meant a product, wherein as beneficial ingredients the same fatty acids of tall oil and of different vegetables are used as above and, if needed, preservatives, solvents and emulsifiers. In an oil-water emulsion the proportion of beneficial fatty acids is 50 - 80 %. Several substances, such as lecithin E322, ethyl cellulose E462, microcrystalline cellulose E 460 or fatty acid mono- and diglycerides can be used as emulsifiers.

As stated above, the curative effect of the products, which are the object of the invention, is based on the synergism of tall oil fatty acids or their derivatives and vegetable oils. The preparations according to the invention may contain 1-90 % by weight of tall oil fatty acids or their derivatives and 99-10 % by weight of vegetable oils. Crude tall oil is a mixture of resin acids and fatty acids, and it also contains small amounts of fatty acid esters, sterols, higher alcohols and hydrocarbons. The exact composition varies depending on the original wood source and the purity grade of the oil. Within this disclosure, with "tall oil", "tall oil fatty acids" and "tall fatty acids" is meant both crude tall oil and the products refined or purified therefrom. For those who suffer from resin allergy, the resin acids of crude tall oil may cause symptoms, and therefore it is more preferable to use purified tall oil wherein the proportion of resin acids is smaller and the proportion of tall oil fatty acids is as high as possible. In the skin care products according to the invention, such a tall oil is preferably used, wherein the proportion of beneficial tall oil fatty acids (TOFA) or tall oil fatty acid derivatives, such as mono-, di- and triglycerides, is about 80-100%, preferably 90-100%, for example about 96%. Fatty acid glycerides are obtained by esterifying tall fatty acids with glycerol. The tall oil fatty acids used in the examples are approximately 96% pure.

Vegetable oils used in the skin care products according to the invention are vegetable oils, which have a high linoleic acid and linolenic acid content, and are selected from the group consisting of sunflower oil, tall seed oil, grape seed oil, safflor oil, flaxseed oil, rapeseed oil, buckthorn oil, blackcurrant seed oil, Siberian pine seed oil, peanut oil, primrose oil, and a mixture of two or more oils. The trend towards products made of natural ingredients is heading to cold-pressed products. Seed oils are the original and best ingredients for skin treatment, but refined products are still being mostly used.

The ratio of tall oil fatty acids or their derivatives and vegetable oils in the preparations according to the invention varies depending on the product form and may be 10:1-1:50, preferably about 1:1-1:10, for example about 1:1 - 1:3, in oily preparations for example 2:1-1:10, preferably about 1:1.

In the tests wherein the preparation according to the invention was used, a 50:50 mixture of tall oil fatty acids and sunflower oil clearly reduced scaling and redness of the skin of psoriasis patients, made thick skin layers thinner and reduced itching (Example 9). The reference substance was a mineral oil-lipogel preparation. Also this preparation clearly reduced the above-mentioned skin symptoms of psoriasis, although to a lesser extent than the mixture of tall oil fatty acids and sunflower oil.

In a comparative blind study wherein a preparation according to the invention or placebo were used, scaling of the skin, thickness, redness and itching were remarkably reduced and lesion was fully recovered in 14 patients and almost fully in six patients (the test group consisted of 20 patients). In the placebo group, only scaling was reduced but otherwise no improvement was detected (Example 10).

The skin care products according to the invention can be prepared in very many different forms, for example as an oil, emulsions, gel, lotion, spray, foam, stick, roll-on, as a so called scalp (liquid) to the scalp and naturally also as a basic cream, depending on the purpose of use and skin condition. The products can thus make up a complete so-called "home pharmacy". They can safely be used individually for each target or as whole body products.

The skin care products according to the invention can be divided for example into three groups:
1. The products, which contain only fatty acids and oils from plants and trees as a mixture.
2. The products which contain oils (fatty acids) from plants and trees, and mineral oils.
3. The products wherein as thickeners of tree oils and vegetable and/or mineral oils, metal derivatives as stearates, *e.g.* magnesium, aluminium, *etc*. can be used.

Further, if needed, the preparations can contain common ingredients used in skin care products, such as stabilizers, emulsifiers, carriers, moisturizing agents, for example carbamides, peeling agents, for example urea, salisylic acid or sulphur, vitamins, for example vitamins A, D, E and C, as well as antioxidants and preservatives, such as benzoic acid, sorbinic acid, citric acid, butyl hydroxy anisol or vitamin E.

An oily preparation according to the invention may contain for example about 35 - about 50% tall oil fatty acids or their derivatives, about 35 - 50% vegetable oils and 0 - 30% above mentioned common ingredients used in skin care products. An oily preparation according to the invention may also contain plenty, even about 90% vegetable oils, especially a mixture of different vegetable oils, and about 1-10% tall oil fatty acids or their derivatives and, if needed, common ingredients used in skin care products.

The emulsions according to the invention may contain for example 1-50% tall fatty acids or their derivatives and 10-50% vegetable oils, and the creams according to the invention for example 1-10% tall fatty acids or their derivatives and 10-40% vegetable oils. The combined amount of tall fatty acids or their derivatives and vegetable oils in the preparations according to the invention may be about 10 -100%, preferably for example 20 - 95%, depending on the preparation. It is to be understood that, depending on the product form, the mutual ratios of the ingredients and the additional conventional ingredients may vary widely within the scope of the invention. However, the preparation according to the invention always comprises tall oil fatty acids or their derivatives and vegetable oil.

The preparations according to the invention may also contain mineral oils and metals. Such skin care products can be prepared as an oil, cream, emulsion and liquid. The base of the cream used for skin care may be prepared from mineral oil (*e*.*g*. paraffin, white oil) and/or vegetable oils (*e.g*. sunflower oil, grape seed oil, rapeseed oil, tall seed oil, peanut oil, safflor oil *etc.*) and tall oil fatty acids (TOFA) or derivatives of tall oil fatty acids, such as mono-, di- and triglycerides. The amount of mineral oil in the preparation may be for example 1-30 % by weight.

The cream base can be thickened by using a metallic soap, which has been prepared from vegetable oils or from palmitic, stearic, oleic, 2-ethylhexadecane acid and a metal salt, such as aluminium, lithium, magnesium and zinc salts. Also solid paraffin can be used for thickening. The amount of thickening agent in the preparation may vary for example between 1-30% by weight.

The UV protection of the cream can be prepared by adding for example titanium dioxide.

As a cream, the metal soap and mineral oil of the preparation form a water-resistant, curing film, which protects the skin from external effects. The protective film can be washed away with warm water and soap. The vegetable oils and tall oil fatty acids of the cream soften and cure the skin and prevent the skin from drying. Titanium dioxide gives an effective shield against detrimental UV radiation. The preparation has been planned for use on the whole body and also under the sun.

The preparations according to the invention can be prepared by as such known methods used in cosmetical and pharmaceutical industry.

When treating severe skin symptoms, the preparation according to the invention, preferably an oil, is thinly smeared with proper rubbing on the symptom-having skin areas 2-3 times a day for about 2-6 weeks, depending on the nature of the disease. The cream can also be used on symptomfree, healthy skin areas, as psoriasis annoyingly tends to come out from the side of the plaques, if only the areas with symptoms are treated. The treatment can be started also with an oil-water emulsion, but the oil is the most efficacious in severe psoriasis. The effect of treatment is generally shown and felt already after 1 week of use. The cornified layer (scale) has almost disappeared.

Redness is treated in the same way, that is by continuing the treatment 1-2 times a day until redness disappears. An alternative to oil is an oil-water emulsion, which may be more pleasant to use.

During a symptomless period, lighter products, such as water-oil emulsion, can be used to maintain the moisture balance of the skin. Water-oil emulsion can also be used during the whole treatment period in addition to oil or oil-water emulsion to maintain the moisture balance of the skin.

The invention is also directed to the use of tall oil fatty acids or their derivatives and vegetable oils selected from the group consisting of sunflower oil, grape seed oil, rapeseed oil, tall seed oil, flaxseed oil, peanut oil, buckthorn oil, blackcurrant seed oil, siberian pine seed oil, safflor oil, primrose oil, and a mixture of two or more oils for the preparation of products for dry and scaling skin, as well as for the preparation of products for the treatment of psoriasis, dermatitis, eczemas or scale. In a method for the treatment and prevention of psoriasis, dermatitis, eczemas or scale, an effective amount of a preparation according to the invention, which comprises tall oil fatty acids or their derivatives and vegetable oils, is spread on the skin

The following examples further illustrate the invention by disclosing the exemplary compositions of the products to be used for skin treatment.-The percentages are given by weight, unless otherwise indicated. Examples 9 and 10 describe treatment results obtained by using the preparations.

### Example 1 Oil

| | |
|---|---|
| Tall oil fatty acids | 42.0 % |
| Sunflower oil | 42.0 |
| Beeswax | 3.5 |
| Titanium dioxide | 3.5 |
| Talc | 1.5 |
| Solid paraffin | 3.5 |
| Vitamin E | 4.0 |
| | 100.0 % |

### Preparation:

1. First, 100 g sunflower oil and 10 g beeswax were warmed and cooled. Cooling resulted in a solid mixture. It was again warmed to fluid.
2. 150 g sunflower oil and 250 g tall oil fatty acids were warmed to 90°Centigrade and poured into sunflower oil and beeswax. Cooling.
   At 55 °C fully flowing, no rise in viscosity.
   35 °C, slight rise in viscosity.
   22 °C, not enough viscosity, we started to whiten with titanium dioxide, first 10 g.
3. Warming again and adding 10 g beeswax and 10 g titanium dioxide. 115 °C, a further 10 g talc were added. Cooling. Not enough viscosity.
4. Warming and adding 20 g solid paraffin. Warming 140 °C. Cooling.
5. Mixing at 30 °C with an emulsifying machine for about one minute (24000 rpm). The product was good at 25°C.

### Example 2 Oil

| | |
|---|---|
| Flaxseed oil | 30.0 % |
| Siberian pine seed oil | 30.0 |
| Primrose oil | 30.0 |
| Tall oil fatty acids | 2.0 |
| Vitamin E | 8.0 |
| | 100.0 % |

### Preparation: Mix cold.

### Example 3 Water-oil emulsion

| | |
|---|---|
| I Lecithin | 4.0 % |
| Safflor oil | 9.0 |
| Tall oil fatty acids | 9.0 |
| Sunflower oil | 9.0 |
| Zinc stearate | 1.0 |
| Tocopheryl (Vitamin E) | 4.0 |
| | |
| II Glycerin 86% | 5.0 |
| Magnesiumsulfate 7 H₂0 | 1.0 |
| Water | 58.0 |
| | 100.0 % |

### Preparation:

1. I The products are mixed and heated 80-85 °C.
   II The products are mixed and heated 80-85 °C.
2. The products I and II are combined and.mixed in the same vessel. The mixture is allowed to cool to 65 - 55 °C, whereby the mixture is homogenized (emulsified) with a fast emulsifying machine with 10 000-20 000 rounds/min. After homogenisation the mixture is cooled to 30 °C. Packing.

This is a product wherein water is in the inner phase, whereby moisture is maintained on the skin for a long time and is not easily evaporated.

### Example 4 Oil-water emulsion

| | |
|---|---|
| I Lecithin | 4.0 % |
| Tall oil fatty acids | 29.0 |
| Sunflower oil | 29.0 |
| Tocopheryl | 4.0 |
| Zinc stearate | 1.0 |
| | |
| II Glycerin 86 % | 5.0 |
| Magnesiumsulfate 7H₂O | 1.0 |
| Water | 27.0 |

Preparation as for the water-oil emulsion in Example 3.

### Example 5 Preparations, which are creamy as to their viscosity

**I**

| | | |
|---|---|---|
| Polyglyceryl-2 Dipolyhydroxystearate | 4% | Emulsifier |
| Polyglyceryl-3 Di-isostearate | 2% | Emulsifier |
| Beeswax | 5% | Thickener |
| Safflor oil | 8% | Beneficial fatty acids |
| Tall oil | 3% | Beneficial fatty acids |
| Sunflower oil | 5% | Beneficial fatty acids |
| Siberian pine seed oil | 5% | Beneficial fatty acids |
| Rapeseed oil | 1% | Beneficial fatty acids |
| Zinc stearate | 1% | Stabilizer |
| Tocopheryl | 2% | Antioxidant |

**II**

| | | |
|---|---|---|
| Glycerin 86% | 5% | Moisturizing agent |
| Magnesium sulfate 7H2O | 1% | Stabilizer |
| Water | 58% | Moisturizing agent |

### Preparation

I. The products are mixed and heated 80-85 °C.
II. The products are mixed and heated 80-85 °C.

The products I and II are combined and mixed in the same vessel.

The mixture is allowed to cool to 65°C-66°C and it is homogenized (emulsified) with a fast 10000-20000 rounds/min machine with emulsifying blades. After homogenisation, the mixture is cooled to about 30 °C. Packing.

This is a product wherein water is in the inner phase whereby moisture is maintained on the skin for a long time and is not evaporated too quickly.

### Example 6 Cream which also contains mineral oil

6% aluminium stearate,
25% mineral oil,
51% tall oil fatty acids and vegetable oils (e.g. sunflower oil, grape seed oil, rapeseed oil, tall seed oil),
15% solid paraffin,
3% titanium dioxide (UV grade, e.g. Kemira UV Titan) and antioxidants and preservatives (*e.g.* benzoic acid, sorbinic acid, citric acid, butylhydroxy anisol BHA, vitamin E).

### Example 7

6% aluminium stearate,
76% tall oil fatty acids and vegetable oils (*e.g.* sunflower oil, grape seed oil, rapeseed oil, tall seed oil),
15% solid paraffin,
3% titanium dioxide and
antioxidants and preservatives (*e.g.* benzoic acid, sorbinic acid, citric acid, butyl hydroxy anisol BHA, vitamin E).

### Example 8 Oil

45% tall oil fatty acids,
5% glycerol,
47-49.1% vegetable oil (*e.g*. sunflower oil, grape seed oil, rapeseed oil, tall seed oil),
0.1-3.0% titanium dioxide and
antioxidatives and preservatives (e.g. benzoic acid, sorbinic acid, citric acid, butyl hydroxy anisol BHA, vitamin E)

### Example 9

Altogether 20 patients with a chronic plaque-type psoriasis were treated with two oil mixtures for six weeks. The products were spread on a certain psoriasis plaque twice a day. Ten patients received a mixture of tall oil fatty acids and linoleic fatty acids (Treatment 1) and ten patients received a corresponding mixture with an added organic aluminium compound (Treatment 2). The psoriasis of the patients had not been treated for four weeks before the beginning of the study, and other similar treatments were not allowed during the study. At the beginning of the study and after six weeks, scaling, redness, the thickness of skin lesion and tickle were evaluated using a four point scale (0 = symptomless, 1 = slight, 2 = medium, 3 = severe). Average values have been calculated for each parameter before and after the test.

**Table 1. Patient data and treatment results**

| | Treatment 1 | Treatment 2 |
|---|---|---|
| Amount of patients | 10 | 10 |
| Male/female | 9/1 | 5/5 |
| Average age | 55.0 | 49.4 |
| Mean duration of psoriasis | 16.7 | 14.6 |

| Location of the plaque followed: | | |
|---|---|---|
| Elbow/knee | 8 | 10 |
| Buttock | 2 | 0 |

| Treatment response: | | |
|---|---|---|
| Fully recovered | 7 | 6 |
| Remarkably recovered | 2 | 3 |
| Poor treatment response | 1 | 1 |
| | | |

| Mean change of single psoriasis symptoms during the treatment: | | |
|---|---|---|
| | | |
| Scaling | 2.5-0.4 | 2.7-0.9 |
| | | |
| Redness | 2.5-0.4 | 2.7-1.0 |
| | | |
| Thickness | 2.4-1.0 | 2.4-1.0 |
| | | |
| Tickle | 1.2-0.3 | 1.2-0.3 |

Treatment 1 proved to be somewhat more effective than Treatment 2. In the treatment group 1 the plaque followed was fully recovered in 7 patients and remarkably recovered for the other two. One patient's plaque had become worse, evidently due to the irritation caused by the oil. In the treatment group 2, the corresponding figures were 6, 3, and 1, respectively.

### Example 10

The study was carried out with 40 patients, using a placebo control. All patients were members of the Finnish Psoriasis Association and had a chronic, stable psoriasis. None of the patients had received treatment for his/her psoriasis during the preceding fortnight. The dosing of creams was randomised so that half the patients received a cream which contained the active agent, and the other half received placebo. The study lasted for four weeks and the cream was used b.i.d. (mornings and evenings during the study period). One lesion located in arm or foot was chosen and followed during the study. The clinical evaluation of the chosen lesion was done before the treatment started and after four weeks by using a four-point scale (0 = symptom free, 1 = slight, 2 = medium, 3 = severe) for the evaluation of scaling, lesion thickness, redness, and tickle. Table 2 shows the changes in the chosen lesion during the study period. The thickness of acanthosis was measured with Dermascan C -apparatus and redness with a photospectrometry.

**Table 2. Changes in the chosen lesions during the experiment period**

| | Study group | | Placebo group | |
|---|---|---|---|---|
| Location of the chosen lesions: | | | | |
| Arm | 15 | | 14 | |
| Foot | 5 | | 6 | |

| | | | | |
|---|---|---|---|---|
| Parameters on week 0 and on week 4: | 0 | 4 | 0 | 4 |
| Scaling, median | 2.3 | 0.3 | 2.2 | 0.5 |
| Thickness, median | 2.7 | 0.5 | 2.7 | 2.5 |
| Redness | 2.9 | 0.5 | 2.8 | 2.8 |
| | | | | |

| Dermascan results: | | | | |
|---|---|---|---|---|
| Acanthosis, median, mm | 0.45 | 0.25 | 0.43 | 0.42 |
| Redness index | 20.3 | 13.7 | 21.0 | 21.2 |
| | | | | |

| Healing: | | | | |
|---|---|---|---|---|
| Fully recovered | 14 | | 0 | |
| Remarkably recovered | 6 | | 0 | |

Scaling was remarkably reduced in both groups, while decreasing of thickness, redness and tickle was detected only in the group, which had received the preparation according to the invention (Study group). In the study group, the chosen lesion had fully recovered in 14 patients and almost recovered in the six others. In the placebo group the healing of the chosen lesion was not found. In the Study group, acanthosis was reduced from 0.45 mm to 0.25 mm and the redness index was reduced from 20.3 to 13.7 (both median). No changes were detected in the placebo group. Opposite changes were not found in either of the groups.

## Claims

1. A skin care product, **characterized in that** it comprises tall oil fatty acids or their derivatives and one or several vegetable oils selected from the group consisting of sunflower oil, grape seed oil, rapeseed oil, tall seed oil, flaxseed oil, peanut oil, buckthorn oil, blackcurrant seed oil, Siberian pine seed oil, safflor oil, primrose oil, and a mixture of two or more oils.

2. The product according to claim 1, **characterized in that** it comprises 1-90 % by weight of tall oil fatty acids or their derivatives and 99-10 % by weight of vegetable oil(s).

3. The product according to claim 1 or 2, **characterized in that** it contains a UV protection agent, for example titanium dioxide.

4. The product according to claim 3, **characterized in that** the amount of the UV protection agent is 0.1-10 % by weight.

5. The product according to any one of claims 1-4, **characterized in that** it is a cream, oil, emulsion, gel, spray, foam, stick, roll-on or scalp.

6. The product according to any one of claims 1-5, **characterized in that** it contains mineral oil, preferably paraffin oil or white oil.

7. The product according to claim 6, **characterized in that** the amount of mineral oil is 1-30 % by weight.

8. The product according to any one of claims 1-7, **characterized in that** it contains a thickener, such as metal soap or solid paraffin.

9. The product according to claim 8, **characterized in that** the amount of thickener is 1-30% by weight.

10. The product according to any one of claims 1-9, **characterized in that** it contains 0.1-10% by weight of glycerol.

11. The use of tall oil fatty acids or their derivatives and vegetable oils selected from the group consisting of sunflower oil, grape seed oil, rapeseed oil, tall seed oil, flaxseed oil, peanut oil, buckthorn oil, blackcurrant seed oil, Siberian pine seed oil, safflor oil, primrose oil and a mixture of two or more oils, for the preparation of skin care products for dry and scaling skin.

12. The use of tall oil fatty acids or their derivatives and vegetable oils selected from the group consisting of sunflower oil, grape seed oil, rapeseed oil, tall seed oil, flaxseed oil, peanut oil, buckthorn oil, blackcurrant seed oil, Siberian pine seed oil; safflor oil, primrose oil and a mixture of two or more oils, for the preparation of preparations for the prevention and treatment of psoriasis, dermatitis, eczemas or scaling.

## Patentansprüche

1. Hautpflegeprodukt, **dadurch gekennzeichnet, dass** es Tallöl-Fettsäuren oder deren Derivate und ein oder mehrere Pflanzenöle, ausgewählt aus Sonnenblumenöl, Traubenkernöl, Rapskernöl, Tallöl, Leinsamenöl, Erdnussöl, Faulbaumöl, Schwarzem Johannisbeerkernöl, Öl der sibirischen Kiefer, Saffloröl, Primelöl und einem Gemisch aus zwei oder mehr Ölen, umfasst.

2. Produkt gemäß Anspruch 1, **dadurch gekennzeichnet, dass** es 1 - 90 Gew.-% Tallöl-Fettsäuren oder deren Derivate und 99 - 10 Gew.-% Pflanzöl(e) umfasst.

3. Produkt gemäß Anspruch 1 oder 2, **dadurch gekennzeichnet, dass** es ein UV-Schutzmittel, zum Beispiel Titandioxid, enthält.

4. Produkt gemäß Anspruch 3, **dadurch gekennzeichnet, dass** die Menge an UV-Schutzmittel 0,1 - 10 Gew.-% beträgt.

5. Produkt gemäß einem der Ansprüche 1 bis 4, **dadurch gekennzeichnet, dass** es eine Creme, ein Öl, eine Emulsion, ein Gel, ein Spray, ein Schaum, ein Stift, ein Roller oder ein Produkt für die Kopfhaut ist.

6. Produkt gemäß einem der Ansprüche 1 bis 5, **dadurch gekennzeichnet, dass** es Mineralöl, vorzugsweise Paraffin oder weißes Öl, enthält.

7. Produkt gemäß Anspruch 6, **dadurch gekennzeichnet, dass** die Menge an Mineralöl 1 - 30 Gew.-% beträgt.

8. Produkt gemäß einem der Ansprüche 1 bis 7, **dadurch gekennzeichnet, dass** es ein Verdickungsmittel, wie eine Metallseife oder festes Paraffin, enthält.

9. Produkt gemäß Anspruch 8, **dadurch gekennzeichnet, dass** die Menge an Verdickungsmittel 1 - 30 Gew.-% beträgt.

10. Produkt gemäß einem der Ansprüche 1 bis 9, **dadurch gekennzeichnet, dass** es 0,1 - 10 Gew.-% Glyerin enthält.

11. Verwendung von Tallöl-Fettsäuren oder deren Derivate und Pflanzenölen, ausgewählt aus Sonnenblumenöl, Traubenkernöl, Rapskernsöl, Tallöl, Leinsamenöl, Erdnussöl, Faulbaumöl, Schwarzem Johannisbeerkernöl, Öl der sibirischen Kiefer, Saffloröl, Primelöl und einem Gemisch aus zwei oder mehr Ölen, zur Herstellung von Hautpflegeprodukten für trockene und schuppende Haut.

12. Verwendung von Tallöl-Fettsäuren oder deren Derivate und Pflanzenölen, ausgewählt aus Sonnenblumenöl, Traubenkernöl, Rapskernsöl, Tallöl, Leinsamenöl, Erdnussöl, Faulbaumöl, Schwarzem Johannisbeerkernöl, Öl der sibirischen Kiefer, Saffloröl, Primelöl und einem Gemisch aus zwei oder mehr Ölen, zur Herstellung von Zubereitungen zur Vorbeugung und Behandlung von Psoriasis, Dermatitis, Ekzemen oder Schuppen.

## Revendications

1. Produit de soins pour la peau, **caractérisé en ce qu'**il comprend des acides gras de tallöl ou leurs dérivés et une ou plusieurs huiles végétales choisies dans l'ensemble constitué de l'huile de tournesol, l'huile de pépins de raisin, l'huile de colza, l'huile de pin, l'huile de lin, l'huile d'arachide, l'huile de nerprun, l'huile de pépins de cassis, l'huile de pin de Sibérie, l'huile de carthame, l'huile de primevère et un mélange de deux ou plus de deux huiles.

2. Produit selon la revendication 1, **caractérisé en ce qu'**il comprend de 1 à 90 % en poids d'acides gras de tallöl ou de leurs dérivés et de 99 à 10 % en poids d'une ou plusieurs huiles végétales.

3. Produit selon la revendication 1 ou 2, **caractérisé en ce qu'**il contient un agent de protection contre les UV, par exemple du dioxyde de titane.

4. Produit selon la revendication 3, **caractérisé en ce que** la quantité d'agent de protection contre les UV est de 0,1 à 10 % en poids.

5. Produit selon l'une quelconque des revendications 1 à 4, **caractérisé en ce qu'**il est une crème, une huile, une émulsion, un gel, un produit à pulvériser, une mousse, un stick, un produit applicable en rouleau ou un produit pour le cuir chevelu.

6. Produit selon l'une quelconque des revendications 1 à 5, **caractérisé en ce qu'**il contient une huile minérale, de préférence de l'huile de paraffine ou de l'huile blanche.

7. Produit selon la revendication 6, **caractérisé en ce que** la quantité d'huile minérale est de 1 à 30 % en poids.

8. Produit selon l'une quelconque des revendications 1 à 7, **caractérisé en ce qu'**il contient un épaississant comme un savon métallique ou de la paraffine solide.

9. Produit selon la revendication 8, **caractérisé en ce que** la quantité d'épaississant est de 1 à 30 % en poids.

10. Produit selon l'une quelconque des revendications 1 à 9, **caractérisé en ce qu'**il contient de 0,1 à 10 % en poids de glycérol.

11. Utilisation d'acides gras de tallöl ou de leurs dérivés et d'huiles végétales choisies dans l'ensemble constitué de l'huile de tournesol, l'huile de pépins de raisin, l'huile de colza, l'huile de pin, l'huile de lin, l'huile d'arachide, l'huile de nerprun, l'huile de pépins de cassis, l'huile de pin de Sibérie, l'huile de carthame, l'huile de primevère et un mélange de deux ou plus de deux huiles, pour la préparation de produits de soins pour la peau pour la peau sèche et desquamante.

12. Utilisation d'acides gras de tallöl ou de leurs dérivés et d'huiles végétales choisies dans l'ensemble constitué de l'huile de tournesol, l'huile de pépins de raisin, l'huile de colza, l'huile de pin, l'huile de lin, l'huile d'arachide, l'huile de nerprun, l'huile de pépins de cassis, l'huile de pin de Sibérie, l'huile de carthame, l'huile de primevère et un mélange de deux ou plus de deux huiles, pour la préparation de préparations pour la prévention et le traitement du psoriasis, d'une dermatite, de l'eczéma ou de la desquamation.
